# FASCICULE DE BREVET EUROPEEN

(11) **EP 1 812 562 B1**
(45) Date de publication et mention de la délivrance du brevet: **27.01.2010**
(21) Numéro de dépôt: 05809271.9
(22) Date de dépôt: 14.10.2005
(51) Int. Cl.: C12N 5/071

(54) **PROCÉDÉ DE PRÉPARATION D'UN SYSTÈME DE CULTURE D'ENDOMÈTRE AUTOLOGUE POUR LA CO-CULTURE ENDOMÈTRE-EMBRYON**
VERFAHREN ZUR HERSTELLUNG EINER AUTOLOGEN ENDOMETRIUMKULTUR FÜR EINE ENDOMETRIUM/EMBRYO-COKULTUR
METHOD FOR PREPARATION OF AN AUTOLOGOUS ENDOMETRIAL CULTURE FOR AN ENDOMETRIUM/EMBRYO COCULTURE

(30) Priorité: 15.10.2004 FR 0410931
(43) Date de publication de la demande: 01.08.2007
(73) Titulaire: Laboratoires Genevrier SA, 06600 Antibes (FR)
(72) Inventeur: VACHER, Dominique Domaine de la Peyrière, F-06250 MOUGINS (FR); CAZALS, Florence, F-06560 VALBONNE (FR); SOLER, Christophe, F-06000 NICE (FR)
(74) Mandataire: Hennion, Jean-Claude
(86) Numéro de dépôt international: PCT/FR2005/002555
(87) Numéro de publication internationale: WO 2006/042947

(56) Documents cités:
- SPANDORFER S D ET AL: "Autologous endometrial coculture in patients with IVF failure: outcome of the first 1,030 cases." THE JOURNAL OF REPRODUCTIVE MEDICINE, vol. 49, no. 6, juin 2004 (2004-06), pages 463-467, XP008050267 ISSN: 0024-7758
- BARMAT L I ET AL: "Autologous endometrial co-culture in patients with repeated failures of implantation after in vitro fertilization-embryo transfer." JOURNAL OF ASSISTED REPRODUCTION AND GENETICS, vol. 16, no. 3, mars 1999 (1999-03), pages 121-127, XP008050265 ISSN: 1058-0468
- BARMAT L I ET AL: "Human preembryo development on autologous endometrial coculture versus conventional medium." FERTILITY AND STERILITY, vol. 70, no. 6, décembre 1998 (1998-12), pages 1109-1113, XP002338116 ISSN: 0015-0282
- NIETO F S ET AL: "The effects of coculture with autologous cryopreserved endometrial cells on human in vitro fertilization and early embryo morphology: A randomized study" JOURNAL OF ASSISTED REPRODUCTION AND GENETICS, vol. 13, no. 5, mai 1996 (1996-05), pages 386-389, XP008050326 ISSN: 1058-0468
- SIMÓN C. ET AL: 'Coculture of human embryos with autologous human endometrial epithelial cells in patients with implantation failure' JOURNAL OF CLINICAL ENDOCRINOLOGY & METABOLISM vol. 84, no. 8, 1999, pages 2638 - 2646
- SIMÓN C. ET AL: 'Localisation of interleukin-1 type I receptor and interleukin-1.beta. in human endometrium throught the mestrual cycle' JOURNAL OF CLINICAL ENDOCRINOLOGY & METABOLISM vol. 77, no. 2, 1993, pages 549 - 555, XP008108050

## Description

La présente invention se rapporte au domaine de la fécondation in vitro et concerne en particulier un procédé de préparation d'un système de culture d'endomètre autologue pour la co-culture endomètre - embryon.

La fécondation extra-corporelle ou fécondation in vitro (FIV) consiste à reproduire au laboratoire ce qui se passe naturellement dans les trompes utérines : la fécondation et les premières étapes du développement embryonnaire. Depuis 1978, année de naissance de Louise Brown jusqu'à présent, cette technique a permis la naissance de centaines de milliers d'enfants dans le monde.

Pour évaluer le taux de réussite des techniques de FIV, plusieurs paramètres sont utilisés dans la littérature. En référence au nombre de ponctions en vue du recueil d'ovocytes, il est possible de prendre en compte :
- les ponctions positives (plus de 95%) ;
- les transferts d'embryons (environ 80%) ;
- le nombre de grossesses cliniques (état de grossesse défini par sac intra-utérin et activité cardiaque) ;
- le nombre d'accouchements ;
- le nombre d'enfants vivants nés.

C'est le nombre d'accouchements avec au moins un enfant normal vivant qui caractérise probablement au mieux le succès des techniques de FIV, ce taux étant de l'ordre de 12 à 15%. Ce taux est fortement dépendant de l'âge des femmes concernées et du nombre des cycles de traitement et du nombre d'embryons transférés.

L'échec d'implantation après FIV peut être d'origine maternelle ou embryonnaire. L'échec d'implantation a été relié, entre autre, à l'aptitude des embryons à atteindre le stade de blastocyste en culture, qui est liée à son tour aux conditions de culture.

Classiquement, après la fécondation in vitro, l'embryon est placé dans des milieux de culture spécifiques synthétiques choisis en fonction du jour de culture (J1 à J6). L'embryon est ensuite replacé dans l'utérus de la patiente à J2 ou J3, c'est-à-dire au stade de 4 ou 8 cellules après culture dans un milieu synthétique. Le taux d'implantation des embryons âgés de 2 ou 3 jours reste faible (de l'ordre de 15% par embryon). Ce faible taux d'implantation peut s'expliquer en partie par l'hypermotricité utérine et la période inappropriée du transfert : en effet, ces embryons sont placés dans un environnement inadéquat puisqu'in vivo, les embryons à ce stade de développement sont encore dans les trompes et que l'embryon n'atteint l'utérus qu'au stade de blastocyste, c'est-à-dire 5 jours après la fécondation.

Des techniques de co-culture (culture d'un embryon jusqu'au stade de blastocyste sur une couche cellulaire servant de support nutritif) ont été proposées pour la première fois chez l'homme en 1989 après que de nombreuses études aient prouvé chez l'animal dès 1962 que cette technique améliorait le taux d'embryons parvenant au stade de blastocyste.

Outre la synchronisation endomètre - embryon, le fait de pouvoir cultiver un embryon jusqu'au stade de blastocyste permet de sélectionner les embryons à pouvoir de développement élevé et d'effectuer un diagnostic préimplantatoire. Par ailleurs, les techniques de co-culture d'embryons permettent d'obtenir des blastocystes de meilleure qualité, par rapport aux blatocystes cultivés dans des milieux synthétiques.

Des techniques de co-culture de l'embryon jusqu'au stade de blastocyste sur des lignées cellulaires animales, comme les cellules Véro provenant d'épithélium de rein de singe vert d'Afrique, par exemple, ont donné des résultats améliorés par rapport au résultats des transferts précoces J2-J3 dans des milieux synthétiques. Cependant, ces cellules animales n'ont fait l'objet d'aucune procédure de validation au regard de la sécurité sanitaire selon la législation en vigueur, dans le but d'une utilisation thérapeutique. L'arrêté du 12.01.1999, fixant les règles de bonnes pratiques en A.M.P. (aide médicale à la procréation) en France, précise que seule la co-culture sur cellules autologues n'induit pas de risques infectieux exogènes.

Devant le principe de précaution, la co-culture a été abandonnée dans les années '98-'99 dans la pratique des laboratoires FIV au profit des milieux synthétiques. De nouveaux milieux synthétiques permettant la culture de l'embryon jusqu'au stade de blastocyste, milieux dits « séquentiels », ont alors fait leur apparition. Aucun de ces milieux n'a cependant pu reproduire l'effet de la co-culture. En effet, des études comparatives entre les blastocystes issus de milieux séquentiels et ceux issus de co-culture montrent que le taux de blastulation est plus important en co-culture (environ 45% contre 30 à 35%), quelque soit la co-culture, et que les blastocystes obtenus sont de meilleures qualité (nombre de blastomères plus important, grade plus élevé, taux de fragmentation diminué). L'effet positif des co-cultures sur le développement de l'embryon est lié aux interactions embryon - cellules, à l'élaboration de cytokines et/ou des facteurs embryotrophiques et trophoblastiques et à la détoxification du milieu de culture par les cellules somatiques.

L'utilisation d'une co-culture de cellules somatiques autologues apparaît dès lors comme la solution idéale permettant des taux d'implantation élevés tout en prévenant la contamination de l'embryon avec des virus exogènes.

Parmi ces cellules somatiques humaines, les cellules d'endomètre occupent une place particulière. Ce sont en effet les cellules physiologiques de l'implantation, ce qui les recommande comme le support idéal de l'embryon lors de la co-culture.

Des systèmes de culture d'endomètre autologue destinés à être utilisés pour la co-culture endomètre - embryon lors de la FIV, qui consistent en une culture de cellules épithéliales purifiées, sont connus. Leur utilisation reste cependant limitée, car la culture de cellules épithéliales des glandes est très fragile, a une durée de vie limitée à 2 à 4 jours, ne supporte qu'un seul passage en culture et est toujours en faible quantité.

Le Demandeur a constaté, et c'est un des mérites de l'invention, que lors de la séparation des différentes fractions cellulaires à partir d'une biopsie d'endomètre, il est possible d'obtenir, en plus d'une première fraction endométriale dite épithéliale (comprenant majoritairement des glandes) et d'une deuxième fraction endométriale dite stromale (comprenant majoritairement des cellules stromales), une troisième fraction endométriale dite mixte qui conduit, après la séparation et la purification des glandes et cellules stromales qui la composent et leur mise en culture, à une culture mixte (de cellules épithéliales et stromales) de qualité, utilisable pour la co-culture endomètre - embryon lors de la FIV.

La présente invention a pour but de proposer un procédé de préparation d'un nouveau système de culture d'endomètre autologue pour la co-culture endomètre - embryon lors de la FIV.

A cet effet et selon un premier aspect, Invention a pour objet un procédé de préparation d'un système de culture d'endomètre autologue destiné à être utilisé pour la co-culture endomètre-embryon lors de la FIV, ledit système de culture comprenant des cellules épithéliales et des cellules stromales, caractérisé en ce que ledit procédé comprend des étapes conduisant à l'obtention d'une première fraction endométriale dite épithéliale, d'une deuxième fraction endométriale dite stromale et d'une troisième fraction endométriale dite mixte comprenant un mélange de glandes et de cellules stromales et en ce qu'il comprend en outre une étape de purification de ladite troisième fraction.

Dans une variante de réalisation, l'étape de purification de la troisième fraction comprend : a) l'obtention de cellules stromales à partir de la troisième fraction et b) l'obtention de glandes à partir de la troisième fraction et c) le mélange des cellules stromales et des glandes ainsi obtenues.

Dans une variante de réalisation, l'étape a) d'obtention de cellules stromales à partir de la troisième fraction endométriale dite mixte comprend les opérations de:
a1. mise en suspension de ladite troisième fraction endométriale dans un milieu de culture approprié conduisant à l'obtention d'une première suspension ;
a2. filtration de ladite première suspension, conduisant à l'obtention d'un premier filtrat et d'une première fraction retenue sur le filtre ;
a3. centrifugation dudit premier filtrat conduisant à l'obtention d'un premier culot contenant des cellules stromales de la troisième fraction endométriale;
a4. reprise dudit premier culot dans un milieu FIV conduisant à l'obtention d'une deuxième suspension de cellules stromales de la troisième fraction endométriale.

Dans une variante de réalisation, l'étape b) d'obtention de glandes à partir de la troisième fraction endométriale dite mixte comprend la filtration de ladite première fraction retenue et la mise en suspension de la deuxième fraction retenue ainsi obtenue, conduisant à l'obtention d'une troisième suspension contenant des glandes de la troisième fraction endométriale, dans un milieu FIV.

Dans une variante de réalisation, l'étape c) consiste dans le mélange desdites deuxième et troisième suspensions pour obtenir une troisième fraction endométriale purifiée.

Le mélange de glandes et de cellules stromales de la troisième fraction endométriale obtenu à l'étape c) est ensuite mis en culture dans un milieu FIV. Les cellules sont maintenues en culture plusieurs jours.

Dans une variante préférée de réalisation, la biopsie d'endomètre est cryoconservée à la réception, auquel cas, environ le jour du déclenchement de l'ovulation chez la patiente, la biopsie est décongelée et soumise au traitement suivant le procédé décrit, puis les glandes et les cellules stromales de la troisième fraction endométriale purifiée sont mises en culture.

Dans une autre variante de réalisation, la biopsie d'endomètre est traitée selon le procédé de l'invention immédiatement après sa réception au laboratoire. Le mélange de glandes et de cellules stromales de la troisième fraction endométriale purifiée ainsi obtenu est mis en culture pendant plusieurs jours ; cette culture cellulaire est cryoconservée et décongelée environ le jour du déclenchement de l'ovulation chez la patiente.

Dans tous les cas, le procédé de préparation d'un système de culture d'endomètre autologue destiné à être utilisé pour la co-culture endomètre-embryon lors de la FIV peut comprendre une étape supplémentaire de conditionnement dudit système de culture d'endomètre autologue au moyen d'un milieu FIV semi solide, en vue du transport vers un laboratoire de Procréation Médicale Assistée.

Selon un deuxième aspect, l'invention concerne un système de culture d'endomètre autologue destiné à être utilisé pour la co-culture endomètre - embryon lors de la FIV, ledit système comprenant une culture de cellules épithéliales et stromales de la troisième fraction endométrite purifiée.

Selon un troisième aspect, l'invention se rapporte à l'utilisation du système de culture d'endomètre autologue décrit pour la préparation de co-cultures endomètre-embryon lors de la FIV.

Selon un quatrième aspect, l'invention a trait à un système de culture d'endomètre autologue prêt à l'emploi, destiné à être utilisé pour la co-culture endomètre - embryon lors de la FIV, caractérisé en ce qu'il comprend :
- une culture de cellules épithéliales et stromales de la troisième fraction endométriale purifiée ;
- un milieu de culture FIV semi solide, pour ladite culture ;
- un emballage stérile pour le système de culture comprenant ladite culture et ledit milieu de culture.

L'invention va maintenant être décrite en détail.

La présente invention a pour objet, selon un premier aspect, un procédé de préparation d'une fraction endométriale mixte purifiée comprenant des cellules stromales et des glandes, à partir d'une biopsie d'endomètre. La biopsie est prélevée sur la patiente en phase lutéale du cycle menstruel. La biopsie est immédiatement placée dans un milieu de culture spécifique au transport et est maintenue à une température comprise entre 4 et 8°C pendant toute la durée du transport. Le transport entre le site de prélèvement et le laboratoire effectuant la culture cellulaire doit se faire le plus rapidement possible ; en général, la biopsie est livrée au laboratoire le lendemain du prélèvement.

L'endomètre est constitué d'un tissu stromal comprenant des cellules mésenchymateuses dites stromales, et des glandes sécrétoires constituées de cellules épithéliales avec une lumière au centre où se déversent les produits de sécrétion.

Le traitement de la biopsie selon l'invention comprend deux étapes de lyse de la biopsie suivies d'une séquence d'étapes de séparation et de purification des deux types cellulaires. Par « lyse de la biopsie d'endomètre» on entend la dissolution de la structure tissulaire de l'endomètre conduisant à l'obtention de cellules (telles que les cellules stromales) ou d'amas de cellules (tels que les glandes sécrétoires) isolés.

La lyse de la biopsie peut s'effectuer par digestion enzymatique, par exemple à l'aide de la collagénase. Dans un autre mode de réalisation, la biopsie peut être lysée mécaniquement.

Le principe de purification des glandes et des cellules stromales est basé sur la différence de taille et de poids entre les cellules stromales petites et légères par rapport aux glandes plus volumineuses et plus lourdes.

A réception, la biopsie est rincée et un contrôle microbiologique du milieu de transport est effectué. Elle est ensuite découpée en petits fragments et les éléments contaminants de l'endomètre (mucus, sang, ...) sont éliminés. Elle est ensuite soit cryoconservée, soit traitée immédiatement pour isoler les différents fractions de cellules.

Les fragments de biopsie sont soumis à une première lyse. Dans une variante de réalisation, la biopsie est digérée à l'aide d'une enzyme, telle que la collagénase.

Le lysat est homogénéisé puis filtré, conduisant à l'obtention d'un filtrat F1 et d'un dépôt D1 riche en glandes (qui correspond à la première fraction endométriale).

Ledit filtrat F1 est centrifugé pendant 5 min environ à 400g environ, permettant l'obtention d'un culot C1.

L'étape de lyse est répétée au moins une fois, comme suit : le dépôt D1 riche en glandes est repris dans un milieu approprié pour obtenir une suspension S1 comprenant, outre des glandes, des fragments d'endomètre non encore digérés.

Par « milieu approprié » on entend toute solution assurant la viabilité et la fonctionnalité optimale d'une population de cellules donnée.

La suspension S1 est soumise à une seconde lyse conduisant à l'obtention d'un second lysat. Celui-ci est homogénéisé puis filtré conduisant à l'obtention d'un filtrat F2 et d'un dépôt D2 riche en glandes.

Si la biopsie d'endomètre est très compacte, une troisième lyse sur le dépôt D2 peut s'avérer nécessaire.

Le filtrat F2 est centrifugé pendant 5 min environ à 400g environ, permettant l'obtention d'un culot C2.

Le culot C1 est repris dans un milieu approprié, conduisant à l'obtention d'une suspension S2, qui servira également pour reprendre le culot C2; une suspension S3 riche en cellules stromales grossièrement isolées est ainsi obtenue ; elle correspond à la deuxième fraction endométriale.

Cette suspension S3 est laissée sédimenter pendant 30 à 60 min environ, conduisant à l'obtention d'un surnageant SN1 riche en cellules stromales et d'un culot C3 comprenant une troisième fraction endométriale dite mixte comprenant un mélange de cellules stromales et de glandes.

De manière caractéristique pour l'invention, il est possible de séparer les glandes et les cellules stromales de cette troisième fraction endométriale, afin de les comptabiliser pour mieux connaître et maîtriser la culture.

### a) Obtention des cellules stromales de la troisième fraction endométriale

Le culot C3 est repris dans un milieu de culture approprié, pour obtenir une suspension S4 (étape a1), qui est ensuite filtrée, conduisant à l'obtention d'un filtrat et d'une fraction retenue sur le filtre FR1 (étape a2).

Le filtrat est centrifugé environ 5 min à 400g, ce qui conduit à l'obtention d'un culot C4 contenant des cellules stromales de la troisième fraction endométriale (étape a3). Le culot C4 est repris dans un milieu FIV conduisant à l'obtention d'une suspension S5 de cellules stromales de la troisième fraction endométriale (étape a4). Sur cette suspension S5 sont alors effectuées des études de viabilité et une numération cellulaire.

Par « milieu FIV » on entend tout milieu de culture utilisé par les laboratoires de procréation lors de la manipulation des gamètes et des embryons.

### b) Obtention de glandes de la troisième fraction endométriale

Par ailleurs, la fraction retenue FR1 est filtrée, permettant de récupérer les glandes. La fraction retenue FR2 ainsi obtenue est ensuite mise en suspension, conduisant à l'obtention d'une suspension S6 contenant des glandes de la troisième fraction endométriale, dans un milieu FIV. Une numération des glandes purifiées est effectuée sur la suspension S6.

### c) Mélanose des glands et des cellules stromales de la troisième fraction endométriale purifiée

Les suspensions S5 et S6 sont ensuite mélangées pour obtenir une troisième fraction endométriale purifiée (étape c).

Le mélange de glandes et de cellules stromales de la troisième fraction endométriale purifiée obtenu à l'étape c) est ensuite mis en culture dans un milieu FIV. Les cellules épithéliales (provenant des glandes) et les cellules stromales sont laissées en cultures pendant quelques jours.

Dans une variante préférée de réalisation, la biopsie d'endomètre est cryoconservée à la réception, auquel cas, environ le jour du déclenchement de l'ovulation chez la patiente, la biopsie est décongelée et soumise au traitement suivant le procédé décrit, puis les glandes et les cellules stromales de la troisième fraction endométriale purifiée sont mises en culture pendant plusieurs jours.

Dans une autre variante de réalisation, la biopsie d'endomètre est traitée selon le procédé de l'invention immédiatement après sa réception au laboratoire. Le mélange de glandes et de cellules stromales de la troisième fraction endométriale purifiée ainsi obtenu est mis en culture pendant plusieurs jours ; cette culture cellulaire est cryoconservée et décongelée environ le jour de la ponction ovocytaire de la patiente.

Dans tous les cas, le procédé de préparation d'un système de culture d'endomètre autologue destiné à être utilisé pour la co-culture endomètre - embryon lors de la FIV peut comprendre une étape supplémentaire de conditionnement dudit système de culture d'endomètre autologue au moyen d'un milieu FIV semi solide ou d'un milieu FIV liquide, en vue du transport vers le Laboratoire de Procréation Médicale Assistée.

Le conditionnement du système de culture d'endomètre autologue comprend au préalable la préparation d'un milieu FIV semi solide à l'aide d'une solution concentrée d'agarose. Un mélange est effectué entre la solution d'agarose et le milieu FIV. Ce mélange est ensuite réparti uniformément dans des boîtes stériles et conservé à 4°C.

Le jour de la fécondation in vitro, la culture de cellules d'endomètre autologue doit être prête à être envoyée au laboratoire de Procréation Médicale Assistée afin d'y placer l'embryon. La condition requise est d'avoir un tapis cellulaire à minimum 60% de confluence.

Dans une variante de réalisation, le milieu FIV semi solide, équilibré au préalable à 37°C et dans une atmosphère contenant 5% de CO₂ pendant au minimum 2h, est déposé sur le tapis de cellules. La plaque est ensuite déposée dans un blister stérile et le tout est scellé par un opercule stérile. L'air à l'intérieur du blister contient 5% de CO₂. Ledit blister est conditionné dans un deuxième blister plus grand. Le conditionnement ainsi obtenu est un double blister.

Dans une autre variante de réalisation, les puits contenant le tapis de cellules sont remplis de 500 µl/puits de milieu de culture liquide (équilibré au préalable à 37°C et dans une atmosphère contenant 5% de CO₂ pendant au minimum 2h), puis ils sont fermés avec des bouchons en silicone. La plaque est ensuite déposée dans un blister stérile et le tout est scellé par un opercule stérile. Le blister est conditionné dans un deuxième blister plus grand. Le conditionnement ainsi obtenu est un double blister.

Le transport vers le laboratoire de Procréation Médicale Assistée est effectué à 27°C environ, dans un temps inférieur à 36h, avec le moins de chocs possible. Dans un carton adapté au transport de marchandises très fragiles est placé un emballage isotherme. A l'intérieur de celui-ci sont placées une ou deux briques de gel eutectique permettant le maintien de la température aux alentours de 27°C. Le blister comprenant la culture de cellules d'endomètre autologue est ensuite déposé sur (ou entre) la (ou les) brique(s) de gel.

Selon un deuxième aspect, l'invention concerne un système de culture d'endomètre autologue destiné à être utilisé pour la co-culture endomètre - embryon lors de la FIV, ledit système comprenant une culture de cellules épithéliales et stromales de la troisième fraction endométriale purifiée.

Dans les procédés de traitement des biopsies d'endomètre connus, ce sont les cellules épithéliales, obtenues par mise en culture des glandes résultant du processus de purification à partir du dépôt D2 (première fraction endométriale), qui sont considérées comme étant les plus intéressantes et elles sont par conséquent utilisées lors de la co-culture endomètre - embryon, comme décrit dans le document Nieto F.S et all. J. Assist. Reprod. Gen 1996, 13(5), 386-389. D'autres documents, par exemple, Spandorfer S.D et al J. Reprod. Med 2004, 49(6), 463-467, divulguent la possibilité d'utiliser lors de la co-culture endomètre-embryon des cellules épithéliales en mélange avec des cellules stromales purifiées à partir du surnageant SN3 (deuxième fraction endométriale). Dans tous les procédés connus, la troisième fraction endométriale dite mixte contenue dans le culot C3 est éliminée.

Or, le Demandeur a constaté que la culture de cellules épithéliales et stromales de la fraction endométriale mixte purifiée est de qualité supérieure à celle des cellules épithéliales purifiées à partir du dépôt D2.

De plus, la culture de cellules épithéliales et stromales de la fraction endométriale mixte purifiée est facilement réalisable, ce qui n'est pas le cas pour la culture de cellules épithéliales provenant de la première fraction endométriale.

Selon un troisième aspect, l'invention se rapporte à l'utilisation d'un des systèmes de culture d'endomètre autologue décrits pour la préparation de cocultures endomètre-embryon lors de la FIV.

Selon un quatrième aspect, l'invention a trait à un système de culture d'endomètre autologue prêt à l'emploi, destiné à être utilisé pour la co-culture endomètre - embryon lors de la FIV, caractérisé en ce qu'il comprend :
- une culture de cellules épithéliales et stromales de la troisième fraction endométriale purifiée ;
- un milieu de culture FIV semi solide, pour ladite culture ;
- un emballage stérile pour le système de culture comprenant ladite culture et ledit milieu.

## Revendications

1. Procédé de préparation, à partir d'une biopsie d'endomètre, d'un système de culture d'endomètre autologue destiné à être utilisé pour la co-culture endomètre - embryon lors de la FIV, ledit système de culture comprenant des cellules épithéliales et des cellules stromales, ledit procédé comprenant des étapes conduisant à l'obtention :
- d'une première fraction endométriale dite épithéliale, contenant les glandes épithéliales provenant du dépôt retenu sur le filtre après une ou plusieurs étapes de lyse du tissu endométrial ;
- d'une deuxième fraction endométriale dite stromale, correspondant aux filtrats cumulés issus des étapes de filtration effectuées après une ou plusieurs étapes de lyse et comprenant comme composante majoritaire des cellules stromales, et
- d'une troisième fraction endométriale dite mixte comprenant un mélange de glandes et de cellules stromales, correspondant au culot de sédimentation de la deuxième fraction,
**caractérisé en ce que** ledit procédé comprend en outre une étape de purification de ladite troisième fraction, conduisant à la séparation des composantes épithéliales et stromales de cette troisième fraction et la mise en culture du mélange de ces composantes purifiées pour obtenir une co-culture mixte.

2. Procédé selon la revendication 1, **caractérisé en ce que** l'étape de purification de ladite troisième fraction endométriale comprend : a) l'obtention de cellules stromales à partir de la troisième fraction et b) l'obtention de glandes à partir de la troisième fraction et c) le mélange des cellules stromales et de glandes ainsi obtenues.

3. Procédé selon la revendication 2, **caractérisé en ce que** l'étape a) comprend les opérations de:
a1. mise en suspension de la troisième fraction endométriale dans un milieu de culture approprié conduisant à l'obtention d'une suspension S4;
a2. filtration de la suspension S4, conduisant à l'obtention d'un filtrat et d'une fraction retenue sur le filtre FR1;
a3. centrifugation dudit filtrat conduisant à l'obtention d'un culot C4 contenant des cellules stromales de la troisième fraction endométriale ;
a4. reprise du culot C4 dans un milieu FIV conduisant à l'obtention d'une suspension S5 de cellules stromales de la troisième fraction endométriale.

4. Procédé selon l'une des revendications 2 ou 3, **caractérisé en ce que** l'étape b) comprend les opérations de :
b1. mise en culture en présence de facteurs favorisant l'attachement des cellules de la fraction retenue FR1 pendant une durée suffisante pour permettre l'adhésion des cellules stromales, conduisant à l'obtention d'un surnageant SN4;
b2. filtration du surnageant SN4 et mise en suspension de la fraction retenue FR2 ainsi obtenue, conduisant à l'obtention d'une suspension S6 contenant des glandes de la troisième fraction endométriale, dans un milieu FIV.

5. Procédé selon les revendications 3 et 4, **caractérisé en ce que** l'étape c) consiste dans le mélange des suspensions S5 et S6 pour obtenir une troisième fraction endométriale purifiée.

6. Procédé selon la revendication 5, **caractérisé en ce qu'**il comprend en outre une étape de mise en culture des glandes et des cellules stromales de la troisième fraction endométriale purifiée.

7. Procédé selon l'une quelconque des revendications 1 à 6, **caractérisé en ce qu'**il comprend une étape supplémentaire de conditionnement dudit système de culture d'endomètre autologue au moyen d'un milieu FIV semi solide, en vue du transport vers un laboratoire de Procréation Médicale Assistée.

8. Procédé selon la revendication 6, **caractérisé en ce que** la biopsie ayant été traitée immédiatement après sa réception au laboratoire, la culture de cellules ainsi obtenue est cryoconservée et décongelée environ le jour de la ponction ovocytaire de la patiente.

9. Procédé selon l'une quelconque des revendications 1 à 6, **caractérisé en ce que**, la biopsie d'endomètre ayant été cryoconservée à la réception, la mise en culture des glandes et des cellules stromales de la troisième fraction endométriale purifiée est réalisée environ le jour de la ponction ovocytaire de la patiente, après décongélation et traitement de ladite biopsie.

10. Procédé selon l'une quelconque des revendications 1 à 9, **caractérisé en ce qu'**il comprend au moins une étape de lyse de la biopsie d'endomètre.

11. Procédé selon la revendication 10, **caractérisé en ce que** la lyse est effectuée avec de la collagénase.

12. Procédé selon la revendication 10, **caractérisé en ce que** la lyse est effectuée de manière mécanique.

13. Système de culture d'endomètre autologue destiné à être utilisé pour la co-culture endomètre - embryon lors de la FIV **caractérisé en ce qu'**il comprend des cellules épithéliales et des cellules stromales de la troisième fraction endométriale purifiée selon le procédé selon l'une des revendications 1 à 12.

14. Utilisation du système de culture d'endomètre autologue, obtenu par la mise en oeuvre du procédé selon les revendications 1 à 12, pour la préparation de co-cultures endomètre - embryon lors de la FIV.

15. Système de culture d'endomètre autologue prêt à l'emploi, destiné à être utilisé pour la co-culture endomètre - embryon lors de la FIV, **caractérisé en ce qu'**il comprend :
- une culture de cellules épithéliales et stromales de la troisième fraction endométriale purifiée selon le procédé selon l'une des revendications 1 à 12;
- un milieu de culture FIV semi solide, pour ladite culture ;
- un emballage stérile pour le système de culture comprenant ladite culture et ledit milieu.

## Claims

1. A method for the preparation, from an endometrial biopsy, of an autologous endometrial culture system intended for use for endometrium-embryo coculture during IVF, said culture system comprising epithelial cells and stromal cells, said method comprising steps yielding:
- a first endometrial fraction referred to as the epithelial fraction which contains the epithelial glands originating from the deposit retained on the filter after the endometrial tissue has undergone one or more lysis steps;
- a second endometrial fraction referred to as the stromal fraction which corresponds to the cumulated filtrate resulting from the filtration steps performed after one or more lysis steps and comprising stromal cells as the majority component; and
- a third endometrial fraction referred to as the mixed fraction which comprises a mixture of glands and of stromal cells corresponding to the sedimentation pellet of the second fraction;
the method being **characterized in that** it further comprises a purification step for purifying said third fraction, thus leading to the separation of the epithelial and stromal components from said third fraction and to the cultivating of the mixture of such purified components in order to yield a mixed coculture.

2. A method according to claim 1, **characterized in that** the step of purification of said third endometrial fraction comprises: a) obtaining stromal cells from the third fraction, b) obtaining glands from the third fraction and c) mixing the stromal cells and glands thus obtained.

3. A method according to claim 2, **characterized in that** step a) comprises the following operations:
a1. placing in suspension the third endometrial fraction in a suitable culture medium, thus yielding a suspension S4;
a2. filtering the suspension S4, thus yielding a filtrate and a fraction retained on the filter FR1;
a3. centrifuging said filtrate, thus yielding a pellet P4 containing stromal cells from the third endometrial fraction; and
a4. taking up the pellet P4 in an IVF medium, thus yielding a suspension S5 of stromal cells from the third endometrial fraction.

4. A method according to claim 2 or claim 3, **characterized in that** step b) comprises the following operations:
b1. cultivating in the presence of factors contributing to the attachment of the cells of the retained fraction FR1 for a sufficient period in order to enable adhesion of the stromal cells, thus yielding a supernatant SN4;
b2. filtering the supernatant SN4 and placing in suspension the retained fraction FR2 thus obtained, thus yielding a suspension S6 containing glands from the third endometrial fraction, in an IVF medium.

5. A method according to claim 3 or claim 4, **characterized in that** step c) consists of mixing the suspensions S5 and S6 to yield a purified third endometrial fraction.

6. A method according to claim 5, **characterized in that** it comprises in addition a step of cultivating the glands and stromal cells from the purified third endometrial fraction.

7. A method according to any one of claims 1 to 6, **characterized in that** it comprises an additional step of packaging said autologous endometrial culture system by means of a semisolid IVF medium, for use during transfer to a medically assisted procreation laboratory.

8. A method according to claim 6, **characterized in that** since the biopsy is treated immediately after it is received by the laboratory, the cell culture thus obtained is cryopreserved and then thawed approximately on the day the patient's oocytes are retrieved.

9. A method according to any one of claims 1 to 6, **characterized in that** since the endometrial biopsy is cryopreserved upon receipt, the glands and stromal cells from the purified third endometrial fraction are cultivated approximately on the day the patient's oocytes are retrieved, after thawing and treatment of said biopsy.

10. A method according to any one of claims 1 to 9, **characterized in that** it comprises at least one step of lysis of the endometrial biopsy.

11. A method according to claim 10, **characterized in that** lysis is carried out with collagenase.

12. A method according to claim 10, **characterized in that** lysis is carried out mechanically.

13. An autologous endometrial culture system intended for use for endometrium-embryo coculture during IVF, **characterized in that** it comprises epithelial cells and stromal cells from the purified third endometrial fraction according to the method according to one of claims 1 to 12.

14. A use of the autologous endometrial culture system, obtained by the implementation of the method according to claims 1 to 12, for the preparation of endometrium-embryo cocultures during IVF.

15. A ready-to-use autologous endometrial culture system intended for use for endometrium-embryo coculture during IVF, **characterized in that** it comprises:
- a culture of epithelial and stromal cells from the purified third endometrial fraction according to the method according to one of claims 1 to 12;
- a semisolid IVF culture medium, for said culture;
- a sterile packaging for the culture system comprising said culture and said medium.

## Patentansprüche

1. Verfahren, um aus einer Endometriumbiopsie ein autologes Endometriumkultursystem herzustellen, welches dazu bestimmt ist, für die Endometrium-Embryo-Cokultivierung bei der IVF verwendet zu werden, wobei das Kultursystem Epithelzellen und Stromazellen umfaßt, wobei das Verfahren Schritte umfaßt, die zur Gewinnung von folgendem führen:
- einer ersten endometrialen, sogenannten epithelialen Fraktion, welche die epithelialen Drüsen enthält, die aus der nach einem oder mehreren Schritt(en) der Lyse des endometrialen Gewebes an dem Filter zurückgehaltenen Ablagerung stammen,
- einer zweiten endometrialen, sogenannten stromalen Fraktion, die den gesammelten Filtraten entspricht, welche aus den nach einem oder mehreren Lyseschritt(en) durchgeführten Filtrationsschritten stammen und als Hauptbestandteil Stromazellen umfassen, und
- einer dritten endometrialen, sogenannten gemischten Fraktion umfassend eine Mischung aus Drüsen und Stromazellen, welche dem Sedimentationsrückstand der zweiten Fraktion entspricht,
**dadurch gekennzeichnet, daß** das Verfahren ferner einen Schritt zur Reinigung der dritten Fraktion umfaßt, was zur Trennung der epithelialen und stromalen Komponenten dieser dritten Fraktion führt, sowie die Kultivierung der Mischung dieser gereinigten Komponenten, um eine gemischte Cokultur zu erhalten.

2. Verfahren nach Anspruch 1, **dadurch gekennzeichnet, daß** der Schritt der Reinigung der dritten endometrialen Fraktion folgendes umfaßt, nämlich a) die Gewinnung von Stromazellen aus der dritten Fraktion und b) die Gewinnung von Drüsen aus der dritten Fraktion sowie c) die Mischung der so gewonnenen Stromazellen und Drüsen.

3. Verfahren nach Anspruch 2, **dadurch gekennzeichnet, daß** Schritt a) die folgenden Arbeitsschritte umfaßt:
a1. Suspendieren der dritten endometrialen Fraktion in einem geeigneten Kulturmedium, was zum Erhalt einer Suspension S4 führt,
a2. Filtrieren der Suspension S4, was zum Erhalt eines Filtrats und einer an dem Filter zurückgehaltenen Fraktion FR1 führt,
a3. Zentrifugieren des Filtrats, was zum Erhalt eines Rückstands C4 führt, der Stromazellen der dritten endometrialen Fraktion enthält,
a4. Aufnehmen des Rückstandes C4 in einem IVF-Medium, was zum Erhalt einer Suspension S5 von Stromazellen der dritten endometrialen Fraktion führt.

4. Verfahren nach einem der Ansprüche 2 oder 3, **dadurch gekennzeichnet, daß** Schritt b) die folgenden Arbeitsschritte umfaßt:
b1. Kultivieren in Anwesenheit von die Bindung der Zellen der zurückgehaltenen Fraktion FR1 begünstigenden Faktoren über einen ausreichenden Zeitraum, um die Adhäsion der Stromazellen zu ermöglichen, was zum Erhalt eines Überstands SN4 führt,
b2. Filtrieren des Überstands SN4 und Suspendieren der so gewonnenen zurückgehaltenen Fraktion FR2, was zum Erhalt einer Drüsen der dritten endometrialen Fraktion enthaltenden Suspension S6 führt, in einem IVF-Medium.

5. Verfahren nach den Ansprüchen 3 und 4, **dadurch gekennzeichnet, daß** Schritt c) im Mischen der Suspensionen S5 und S6 besteht, um eine gereinigte dritte endometriale Fraktion zu erhalten.

6. Verfahren nach Anspruch 5, **dadurch gekennzeichnet, daß** es ferner einen Schritt zum Kultivieren der Drüsen und der Stromazellen der gereinigten dritten endometrialen Fraktion umfaßt.

7. Verfahren nach einem der Ansprüche 1 bis 6, **dadurch gekennzeichnet, daß** es einen zusätzlichen Schritt zum Verpacken des autologen Endometriumkultursystems mit Hilfe eines halbfesten IVF-Mediums im Hinblick auf den Transport zu einem Labor für medizinisch unterstützte Fortpflanzung umfaßt.

8. Verfahren nach Anspruch 6, **dadurch gekennzeichnet, daß** wenn die Biopsie unmittelbar nachdem sie im Labor in Empfang genommen wurde, behandelt worden ist, die so erhaltene Zellkultur kryokonserviert und etwa am Tag der Eizellenpunktion bei der Patientin aufgetaut wird.

9. Verfahren nach einem der Ansprüche 1 bis 6, **dadurch gekennzeichnet, daß** wenn die Endometriumbiopsie beim Empfang kryokonserviert worden ist, die Kultivierung der Drüsen und der Stromazellen der gereinigten dritten endometrialen Fraktion etwa am Tag der Eizellenpunktion bei der Patientin, nach dem Auftauen und der Behandlung der Biopsie durchgeführt wird.

10. Verfahren nach einem der Ansprüche 1 bis 9, **dadurch gekennzeichnet, daß** es wenigstens einen Schritt zur Lyse der Endometriumbiopsie umfaßt.

11. Verfahren nach Anspruch 10, **dadurch gekennzeichnet, daß** die Lyse mit Kollagenase durchgeführt wird.

12. Verfahren nach Anspruch 10, **dadurch gekennzeichnet, daß** die Lyse mechanisch durchgeführt wird.

13. Autologes Endometriumkultursystem, welches dazu bestimmt ist, für die Endometrium-Embryo-Cokultivierung bei der IVF verwendet zu werden, **dadurch gekennzeichnet, daß** es Epithelzellen und Stromazellen der gereinigten dritten endometrialen Fraktion gemäß dem Verfahren nach einem der Ansprüche 1 bis 12 umfaßt.

14. Verwendung des durch Durchführen des Verfahrens nach den Ansprüchen 1 bis 12 erhaltenen autologen Endometriumkultursystems für die Herstellung von Endometrium-Embryo-Cokulturen bei der IVF.

15. Einsatzbereites autologes Endometriumkultursystem, das dazu bestimmt ist, für die Endometrium-Embryo-Cokultivierung bei der IVF verwendet zu werden, **dadurch gekennzeichnet, daß** es folgendes umfaßt:
- eine Kultur von Epithel- und Stromazellen der gereinigten dritten endometrialen Fraktion gemäß dem Verfahren nach einem der Ansprüche 1 bis 12,
- ein halbfestes IVF-Kulturmedium für die Kultur,
- eine sterile Verpackung für das Kultursystem umfassend die Kultur und das Medium.
